# EUROPÄISCHE PATENTSCHRIFT

(11) **EP 0 446 872 B1**
(45) Veröffentlichungstag und Bekanntmachung des Hinweises auf die Patenterteilung: **21.09.1994**
(21) Anmeldenummer: 91103763.8
(22) Anmeldetag: 12.03.1991
(51) Int. Cl.: C07D 213/61

(54) **Verfahren zur Herstellung von 2,3-Dichlor-5-acetylpyridin**
Process for the preparation of 2,3-dichloro-5-acetylpyridine
Procédé de préparation de 2,3-dichloro-5-acétylpyridine

(30) Priorität: 14.03.1990 CH 834/90
(43) Veröffentlichungstag der Anmeldung: 18.09.1991
(73) Patentinhaber: LONZA AG, CH-3945 Gampel/Wallis (CH)
(72) Erfinder: Kuo, David L., Dr., Brig, (Wallis) (CH)
(74) Vertreter: Weinhold, Peter, Dr.

(56) Entgegenhaltungen:
- EP-A- 0 299 277
- EP-A- 0 341 478
- US-A- 4 358 455
- Louis F.Fieser,Mary Fieser: "Reagents for Organic Synthesis" vol.1 1967, JOHNWILEY AND SONS,INC., New York

## Beschreibung

Die vorliegende Erfindung betrifft ein Verfahren zur Herstellung von 2,3-Dichlor-5-acetylpyridin. 2,3-Dichlor-5-acetyl-pyridin ist ein Zwischenprodukt zur Herstellung von Pyridyl-ethanolaminen, die Wirkstoffe zur Förderung der Leistung von Tieren sind.

Es ist bekannt (EP 0 341 478 A2) 2,3-Dichlor-5-acetylpyridin aus 5,6-Dichlornikotinsäurehalognide mit Methylmagnesiumhalogenid in Gegenwart von Eisenkatalysatoren wie Eisen(II)-chlorid oder Tris-(2,4-pentandionato)-eisen herzustellen. Die Ausbeuten, bezogen auf 5,6-Dichlornikotinsäurehalogenid liegen bei diesem bekannten Verfahren bei rund 50%.

Ziel der vorliegenden Erfindung ist es ein Verfahren zur Verfügung zu stellen, das wesentlich höhere Ausbeuten erzielt. Erfindungsgemäss wird dies, nach einem Verfahren wie in Anspruch 1 beansprucht, erreicht.

Vorzugsweise geht man von 5,6-Dichlornikotinsäurechlorid aus, das man mit Magnesiumchlorid in Gegenwart einer katalytischen Menge Cu/CuCl umsetzt.

Selbstverständlich können auch andere 5,6-Dichlornikotinsäurehalogenide wie z.B. - Bromid oder Jodid und andere Methylmagnesiumhalogenide, wie z.B. Methylmagnesiumbromid, eingesetzt werden.

Pro Mol Nikotinsäurehalogenid werden vorteilhaft 0,8 bis 1,2 Mol Methylmagnesiumhalogenid und 1 bis 10 Mol% Katalysatoren eingesetzt.

Die Umsetzung wird in Gegenwart eines inerten Verdünnungsmittels wie Ether, z.B. Diethyl- und Dibutylether, Glycoldimethylether, Dioxan, einem aliphatischen oder aromatischen Kohlenwasserstoff, wie Pentan, Hexan, Heptan, Cyclohexan, Benzol, Toluol, Benzin, Petrolether durchgeführt. Vorzugsweise wird Tetrahydrofuran eingesetzt.

Die Umsetzungstemperatur liegt zweckmässig bei -70°C bis +30°C, vorzugsweise bei -30°C bis 0°C.

Die Aufarbeitung der Umsetzungsgemische erfolgt wie es bei Grignard-Reaktionen üblich ist.

### Beispiel

### Herstellung von 2,3-Dichlor-5-acetylpyridin

In einem Rundkolben wurden 25,4 g (120,7 mmol) 5,6-Dichlornikotinsäurechlorid, 79 mg (1,2 mmol) Kupferpulver und 119 mg (1,2 mmol) Kupfer (I)-chlorid vorgelegt. Nach dem Verdrängen der Luft durch Stickstoff wurden 200 ml trockenes Tetrahydrofuran zugegeben und die Mischung auf -30°C abgekühlt. Bei dieser Temperatur wurden während 1,5 Std. 40 ml einer 3 molaren Lösung von Methylmagnesiumchlorid (120,4 mmol) in Tetrahydrofuran zugetropft. Das Reaktionsgemisch wurde anschliessend noch 1 Std. weiter gerührt und dann mit 25 ml einer gesättigter Kochsalzlösung gequenscht. Nach der üblichen Aufarbeitung (Extraktion mit Ethylacetat) erhielt man 22,4 g Rohprodukt, das nach GC einen Gehalt von 96,3% 2,3-Dichlor-5-acetylpyridin und 3,5% des entsprechenden Carbinols aufwies. Dies entspricht einer Ausbeute an 2,3-Dichlor-5-acetylpyridin von 94%.

## Patentansprüche

1. Verfahren zur Herstellung von 2,3-Dichlor-5-acetylpyridin durch Umsetzen von 5,6-Dichlornikotinsäurehalogenid mit Methylmagnesiumhalogenid in einem inerten organischen Verdünnungsmittel, dadurch gekennzeichnet, dass man die Umsetzung in Gegenwart von Cu/Cuhalogenid als Katalysator durchführt.

2. Verfahren nach Patentanspruch 1, dadurch gekennzeichnet, dass man die Umsetzung von Dichlornikotinsäurechlorid mit Methylmagnesiumchlorid in Gegenwart von Cu/CuCl als Katalysator durchführt.

## Claims

1. A process for the preparation of 2,3-dichloro-5-acetyl pyridine by reacting 5,6-dichloronicotinic acid halide with methylmagnesium halide in an inert organic diluent, characterized in that the reaction is conducted in the presence of Cu/Cu-halide as a catalyst.

2. The process according to Claim 1 , characterized in that the reaction of dichloronicotinic acid chloride with methylmagnesium chloride is conducted in the presence of Cu/CuCl as a catalyst.

## Revendications

1. Procédé pour la préparation de 2,3-dichloro-5-acétylpyridine par réaction d'halogénure de l'acide 5,6-dichloronicotinique avec un halogénure de méthylmagnésium dans un agent diluant organique inerte, caractérisé en ce que l'on conduit la réaction en présence de Cu/halogénure de Cu comme catalyseur.

2. Procédé selon la revendication 1, caractérisé en ce que l'on conduit la réaction du chlorure de l'acide dichloronicotinique avec du chlorure de méthylmagnésium en présence de Cu/CuCl comme catalyseur.
